(19) Europäisches Patentamt
European Patent Office
Office européen des brevets

(11) Publication number: **0 638 573 A1**

(12) **EUROPEAN PATENT APPLICATION**

(21) Application number: **93112822.7**

(51) Int. Cl.$^6$: **C07D 501/36**, C07D 501/12

(22) Date of filing: **10.08.93**

(43) Date of publication of application:
**15.02.95 Bulletin 95/07**

(84) Designated Contracting States:
**AT BE CH DE DK ES FR GB GR IE IT LI LU MC NL PT SE**

(71) Applicant: **LUCKY LTD.**
**20, Yoido-dong,**
**Yongdungpo-ku**
**Seoul 150-721 (KR)**

(72) Inventor: **Kim, Sung-Hag**
**254-49, Suyou-Dong,**
**Dobong-ku**
**Seoul (KR)**
Inventor: **Kim, Jun**
**Lucky Hana APT. 107-1503, 153**
**Shinsung-dong**
**Youseong-ku, Daejon (KR)**
Inventor: **Yeo, Jae-Hong**
**Lucky Hana APT. 107-1505, 153**
**Shinsung-dong**
**Youseong-ku, Daejon (KR)**
Inventor: **Ok, Jong-Hoa**
**Lucky Apt.9-104,**
**391-42, Doryong-Dong**
**Youseong-Ku,**
**Daejeon (KR)**
Inventor: **Jo, Nag-Sug**
**Clover Apt,**
**102-1305, Samchun-Dong**
**Seo-Ku,**
**Daejon (KR)**

(74) Representative: **Patentanwälte Grünecker, Kinkeldey, Stockmair & Partner**
**Maximilianstrasse 58**
**D-80538 München (DE)**

(54) **Crystalline hydrates of cephalosporin and process for preparation thereof.**

(57) The present invention relates to a crystalline hydrate of 7-[(Z)-2-(2-aminothiazole-4-yl)-2-(2-carboxylprop-2-oxyimino) acetamido]-3-(5-methyl-1,4,6-triaminopyrimidinium-2-yl) thiomethyl-3-cephem-4-carboxylate and a composition comprising said compound as an active ingredient. Also provided herein is a process for preparation of the compound which comprises the steps of: dissolving the compound in an amorphous form or its salt in an acidic or basic solution; adjusting the pH of the solution to a range from 1.5 to 5.5 by adding a basic or acidic solution; precipitating the compound in a crystalline form in the solution; and recovering said crystalline hydrate of the compound.

Figure 1

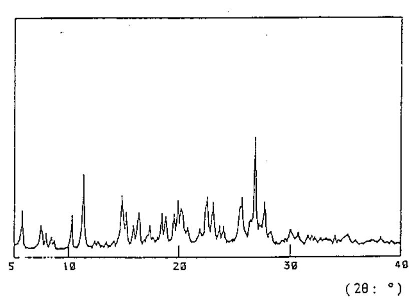


X-ray diffraction diagram (Cu-Kα, 32 KV, 15 mA)

EP 0 638 573 A1

Field of the Invention

The present invention relates to a crystalline hydrate of 7-[(Z)-2-(2-aminothiasole-4-yl)-2(2-carboxyprop-2-oxyimino)acetamido]-3-(5-methyl-1,4,6-triamino-pyrimidium-2-yl)thiomethyl-3-cephem-4-carboxylate which may be represented by formula(I), useful as an antibiotic, and a process for the preparation thereof.

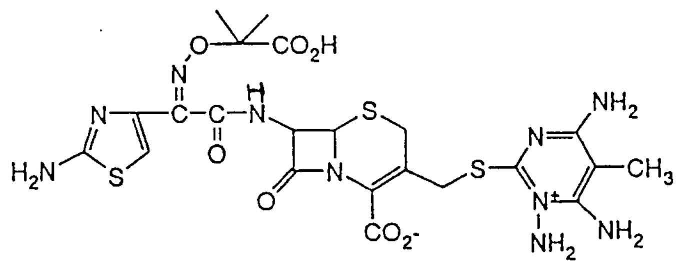

(I)

Description of the Prior Art

European Patent Publication No. 397,511 discloses a cephalosporin of the above formula (I) in an amorphous state which has a potent antibiotic efficacy against a wide spectrum of pathogenic bacteria including Gram positive and Gram negative bacteria producing β-lactamase.

However, the amorphous form of said cephalosporin compound as disclosed in the above-mentioned EP patent publication is unstable itself; and, therefore, when exposed to a high temperature or stored at a room temperature for a long time, β-lactam ring of the cephalosporin of formula(I) is susceptible to a hydrolysis which may result in the decomposition of the ring or compound.

Summary of the Invention

Accordingly, it is an object of the present invention to provide a crystalline hydrate of the formula(I) cephalosporin, possessed with an excellent stability and prolonged shelf-life.

As another object of the present invention, there is provided a process for preparing said crystalline hydrate of the formula(I) cephalosporin.

Still another object of the present invention lies in providing a pharmaceutical composition comprising a therapeutically effective amount of said crystalline hydrate and a physiologically acceptable carrier.

Brief Description of the Drawing

Fig. 1 depicts an X-ray diffraction diagram for the crystalline hydrate of formula(I) cephalosporin.

Detailed Description of the Invention

In accordance with the present invention, it has been found that crystalline hydrates of cephalosporin of formula(I) have surprisingly effective antibiotic activities against a wide spectrum of pathogenic bacteria including Gram positive and negative bacteria; and further have an excellent stability even under severe conditions.

A standard crystalline hydrate form of the compound is hexahydrate that normally exits at a temperature of 20°C and a relative humidity of 33%; however, tri- to hepta-hydrates of the compound are convertible among themselves at a temperature ranging from 10°C to 60°C and at a relative humidity ranging from 10 to 80%, without changing the properties of the compound. This conversion is completely reversible; and, therefore, any desired form of hydrates can be obtained by controlling, e.g., the surrounding temperature and humidity thereof.

The crystalline hydrates of the formula(I) cephalosporin in accordance with the present invention may be prepared by the steps of: (a) dissolving the amorphous compound or its salt of formula(I) in an acidic or basic solution in an amount ranging from 5% to 60% by weight, preferably from 10 to 30% by weight; (b) adjusting the pH of said solution to a range from 1.5 to 5.5, preferably from 2.5 to 4.0, by adding a basic or acidic solution; (c) precipitating the compound in a crystalline form from said solution; and (d) recovering

said crystalline hydrate of the compound.

Representative solvents which may be employed in preparing the acidic or basic solution used in step-(a) above include commercial grade distilled water and water-miscible organic solvents such as acetone.

During the process adjusting the pH of the solution in step(b) above, when the solution is basic, an organic and/or inorganic acid is introduced into the solution. Representative organic acids which may be employed therefor include formic, acetic, phthalic, trifluouroacetic, para-toluenesulfonic acids and the like; and useful examples of inorganic acids include hydrochloric, sulfuric, nitric, phosphoric, bromic acids and the like.

When the solution is acidic, an organic and/or inorganic base may be added into the solution. Representative organic bases include procaine, phenyl benzylamine, dibenzylethylenediamine, ethanolamine, diethanolamine and the like; and exemplary inorganic bases include hydroxide, carbonate, $HCO_3^-$ or ammonium hydroxide of alkaline metals or alkaline earth metals.

During the precipitation in step(c) above, the temperature of the solution is preferably maintained at a range from 0 °C to 60 °C, more preferably from 10 °C to 35 °C to form the crystalline structure.

Further, in order to accelerate the crystallization, crystalline fractions of the compound, independently obtained, may be added to the solution as a "seed."

In step(d) above, the precipitate is filtered, washed and dried in accordance with a conventional method to obtain crystalline hydrates of the formula(I) cephalosporin.

The peaks of the crystalline hydrates of the formula(I) cephalosporin in accordance with the present invention are the same as those of the noncrystalline formula(I) compound, as verified through the analyses of infrared spectroscopy(IR), nuclear magnetic resonance(NMR), mass spectroscopy and liquid chromatography(HPLC). However, polarization microscopic and powder X-ray diffraction analyses reveal the crystalline form of the compound to be different from the noncrystalline compound, as shown in Fig 1.

Table 1 given below represents the characteristic values of the compound in a crystalline form measured by powder X-ray diffraction, wherein d represents the distance between crystal faces and I/Io represents the relative intensity.

Table 1

| X-ray Diffraction Data on Crystalline State of Formula(I) Compound | | | | | |
|---|---|---|---|---|---|
| d(Å) | intensity | d(Å) | intensity | d(Å) | intensity |
| 15.38 | M | 11.87 | W | 11.15 | VW |
| 10.52 | VW | 10.20 | tr | 8.56 | M |
| 7.78 | M | 7.14 | tr | 6.96 | tr |
| 6.10 | tr | 5.96 | M | 5.79 | M |
| 5.56 | W | 5.39 | M | 5.08 | W |
| 4.80 | W | 4.71 | W | 4.53 | W |
| 4.45 | M | 4.39 | M | 4.27 | VW |
| 3.94 | M | 3.85 | M | 3.76 | VW |
| 3.70 | VW | 3.49 | M | 3.47 | M |
| 3.38 | W | 3.32 | S | 3.27 | W |
| 3.22 | M | 3.15 | VW | | |
| S: strong, M: middle, W: weak, VW: very weak, tr: trace | | | | | |

The crystalline hydrates of the formula(I) cephalosporin so obtained may be mixed with a pharmaceutically acceptable carrier to provide a pharmaceutical composition. Representative carriers useful in the present invention include: lactose, dextrose, sucrose, sorbitol, mannitol, starches, gum acacia, calcium phosphate, alginates, tragacanth, gelatin, calcium silicate, microcrystalline cellulose, methylhydroxybenzoates, propylhydroxybenzoates, talc, magnesium stearate, mineral oil and the like. The formulation may additionally include lubricating, wetting, sweetening, flavoring, emulsifying and suspending agents, preservatives and the like. The active ingredient present in the composition may range from 0.1% to 99.9% by weight thereof.

The following Examples are given for the purpose of illustration only and are not intended to limit the scope of the invention.

Preparation Example: preparation of amorphous sodium 7-[(Z)-2-(2-aminothiazole-4-yl)-2(2-carboxyprop-2-oxyimino)acetamido]-3-(5-methyl-1,4,6-triaminopvrimidium-2-yl)thiomethyl-3-cephem-4-carboxylate

Amorphous 7-[(Z)-2-(2aminothiazole-4-yl)-2-(2-carboxyprop-2-oxyimino)acetamido]-3-(5-methyl-1,4,6-triamino-pyrimidinium-2-yl)thiomethyl-3-cephem-4-carboxylate was prepared in accordance with the procedure disclosed in the above-mentioned EP publication for the further use.

6.4g of said compound was added to 200ml of DMF(dimethyl formamide)solution; and 1.1N sodium-2-ethylhexanate in 10ml of DMF solution was added thereto slowly. After stirring the mixture for 1 hour at a room temperature, 90ml of ether was added thereto; and the mixture was further stirred for additional 30 minutes. The resultant precipitate was filtered, washed with 20ml of ether, and dried to obtain 6.3g of a compound. The compound was identified as the title compound in an amorphous state by using X-ray diffraction analysis.

The compound was further identified as follows:

Molecular formula: $C_{22}H_{25}N_{10}O_7S_3 \cdot Na$

Sodium content:

found: 3.3% calculated: 3.5%

Example 1

Step 1)

To 50 ml of distilled water was added 5g of amorphous 7-[(Z)-2-(2-aminothiazole-4-yl)-2-(2-carboxyprop-2-oxyimino)acetamido]-3-(5-methyl-1,4,6-triaminopyrimidinium-2-yl)thiomethyl-3-cephem-4-carboxylate obtained in the above Preparation Example at 30°C; and 10ml of 0.5N sodium hydroxide solution was dropwise added thereto with stirring. Under continuous stirring, the pH of the solution was adjusted to 4.0 with the addition of 1N hydrochloric acid at a rate of 0.1ml/min; and the solution was further stirred for 1 hour. The pH of the solution was adjusted to 3.5 by adding 1N hydrochloric acid and stirred for 1 hour. The precipitate was filtered, washed with 50ml of distilled water and dried to give 3.8g of a pale yellow crystalline compound.

Step 2)

The crystalline compound obtained in step 1 was equilibrated at the relative humidity of 33% and temperature of 20°C; and was further identified as:

Molecular formula: $C_{22}H_{26}N_{10}O_7S_3 \cdot 6H_2O$

Water content: (ASTM E203-75)

found: 14.3% calculated: 14.5%

In terms of powder X-ray diffraction pattern, the peaks of the compound obtained herein were different from those of the amorphous cephalosporin obtained in Preparation Example described above; however, their IR and NMR spectra were the same.

Example 2

Step 1)

3g of amorphous 7-[(Z)-2-(2-aminothiazole-4-yl)-2-(2-carboxyprop-2-oxyimino)acetamido]-3-(5-methyl-1,4,6-triaminopyrimidinium-2-yl)thiomethyl-3-cephem-4-carboxylate obtained in the above Preparation Example was added to 30ml of distilled water at 30°C; and 8ml of 1N hydrochloric acid solution was added thereto slowly under stirring. 2.5N sodium hydroxide solution was added to the solution at a rate of 0.05ml/min for 8 minutes, and the mixture was stirred for 1 hour. The same sodium hydroxide solution was further added to the resultant solution at a rate of 0.1ml/min for 28 minutes, which was stirred for 2 hours. After the completion of stirring, the precipitate was filtered, washed with 30 ml of distilled water and dried to provide 2.5g of a pale yellow crystalline compound.

Step 2)

The crystalline compound obtained in step 1 was equilibrated at the relative humidity of 33% and temperature of 20°C; and was further identified as:

Molecular formula: $C_{22}H_{26}N_{10}O_7S_3 \cdot 6H_2O$

Water content:

found: 14.2% calculated: 14.5%

Example 3

Step 1)

0.2ml of 2.5N sodium hydroxide solution was added to 20ml of distilled water at 5°C; and 0.5g of amorphous 7-[(Z)-2-(2-aminothiazole-4-yl)-2-(2-carboxyprop-2-oxyimino)acetamido]-3-(5-methyl-1,4,6-triamino-pyrimidinium-2-yl)thio-methyl-3-cephem-4-carboxylate obtained in the above Preparation Example was dissolved in the solution completely under stirring. The pH thereof was adjusted to 5.2 with the addition of 0.1N p-toluene-sulfonic acid.

A crystalline seed previously prepared was added to the above solution, followed by stirring. 0.1N p-toluene-sulfonic acid was added to the solution to adjust the pH thereof to 3.3. While maintaining the pH, the solution was stirred for 1 hour, and the crystalline precipitate was filtered and dried to obtain 0.42g of a pale yellow crystalline compound.

Step 2)

The crystalline compound obtained in Step 1 was equilibrated at the relative humidity of 33% and temperature of 20°C; and was further identified as follows:

Molecular formula: $C_{22}H_{26}N_{10}O_7S_3 \cdot 6H_2O$

Water content:

found: 14.7% calculated: 14.5%

Example 4

Step 1)

5ml of 0.5N hydrochloric acid was added to 20 ml of distilled water at 20°C; and 0.5g of amorphous 7-[(Z)-2-(2-aminothiazole-4-yl)-2-(2-carboxylprop-2-oxyimino)acetamido]-3-(5-methyl-1,4,6-triaminopyrimidinium-2-yl)thiomethyl-3-cephem-4-carboxylate obtained in the above Preparation Example was added thereto; and the solution was stirred to completely dissolve the compound. While stirring the solution continuously, 0.5N ethanolamine solution was slowly added thereto to adjust the pH thereof to 1.5.

A crystalline seed previously prepared was added to the resultant solution, and its pH was adjusted to 3.3 with the addition of the above ethanolamine solution slowly. The precipitate was filtered and dried to

yield a 0.40g of a pale yellow crystalline compound.

Step 2)

The crystalline compound obtained in Step 1 was equilibrated at the relative humidity of 12% and temperature of 20 ° C; and further identified as follow:

Molecular formula: $C_{22}H_{26}N_{10}O_7S_3 \cdot 5H_2O$

Water content:

found: 12.7% calculated: 12.4%

Example 5

One(1)g of amorphous sodium 7-[(Z)-2-(2-aminothiazole-4-yl)-2-(2-carboxylprop-2-oxyimino)acetamido]-3-(5-methyl-1,4,6-triaminopyrimidinium-2-yl)thiomethyl-3-cephem-4-carboxylate prepared in the above Preparation Example was added to 20ml of distilled water and was allowed to dissolve completely. While stirring the mixture continuously, 1N hydrochloric acid was slowly added thereto to adjust the pH thereof to 3.5; and the resultant solution was stirred for 1 hour. The precipitate was filtered and dried to yield 0.7g of a pale yellow crystalline compound, which was further identified as:

Molecular formula: $C_{22}H_{26}N_{10}O_7S_3 \cdot 6H_2O$

Water content:

found: 14.6% calculated: 14.5%

Example 6: Efficacy Test

Antibiotic activities of the crystalline compound obtained in Example 1 were evaluated by way of determining the minimum inhibitory concentration(MIC) for the microorganisms listed in Table 2 in vitro.

The MSC was determined in accordance with the doubling dilution method(NCCLS). After the test compound was dispersed in a Muller-Hinton agar medium, the medium was inoculated with 2μl of a test strain having $10^7$ CFU/ml and, then, incubated for 20 hours at 37 ° C.

For comparison, antibiotic abilities of the amorphous formula(I) cephalosporin obtained in Preparation Example were also determined as above; and are shown in Table 2.

Table 2

| Antibiotic Efficacy of the Compounds of Example 1 and Preparation Example(MIC, $\mu$g/ml) | | | |
|---|---|---|---|
| Strain | | MIC(mcg/ml) | |
| | | Test Compound | Amorphous Compound |
| Bacillus cereus | ATCC11778 | 64 | 128 |
| Bacillus megaterium | ATTCC9885 | 0.13 | - |
| Micrococcus luteus | ATCC9341 | 0.25 | 0.5 |
| Staphylococcus aureus | ATCC6538p | 4 | 4 |
| Staphylococcus aureus | ATCC10537 | 2 | 2 |
| Streptococcus epidermidis | ATCC12228 | 1 | 2 |
| Streptococcus faecalis | ATCC29212 | >128 | >128 |
| Acinetobacter calcoaceticus | ATCC15473 | 1 | 4 |
| Citrobacter freundii | ATCC8090 | 0.016 | 0.063 |
| Enterobacter aerogenes | ATCC29751 | 0.25 | 1 |
| Enterobacter cloacae | ATCC27508 | <=0.008 | 0.016 |
| Escherichia coli | ATCC10536 | 0.031 | 0.063 |
| Escherichia coli | ATCC25922 | 0.031 | 0.13 |
| Klebsiella pneumoniae | ATCC10031 | 0.016 | 0.063 |
| Morganella morganii | ATCC8076h | <=0.008 | <=0.008 |
| Proteus mirabilis | ATCC25933 | 0.016 | 0.031 |
| Proteus vulgaris | ATCC6059 | 0.031 | 0.063 |
| Providencia rettgeri | ATCC9250 | <=0.008 | <=0.008 |
| Salmonella typhimurium | ATCC14028 | 0.063 | 0.13 |
| Serratia marcescens | ATCC27117 | 0.063 | 0.13 |
| Shigella flexneri | ATCC11836 | 0.016 | 0.031 |
| Shigella sonnei | ATCC11060 | 0.031 | 0.063 |
| Pseudomonas aeruginosa | ATCC25619 | 0.5 | 2 |
| Pseudomonas aeruginosa | ATCC27853 | 0.5 | 2 |
| Pseudomonas aeruginosa | ATCC10145 | 1 | 2 |

Example 7: Heat-Stability Test

The crystalline hydrate of the compound obtained in Example 1 and the amorphous cephalosporin obtained in Preparation Example were stored at 50°C. After the respective periods of one week and two weeks, the heat stability of the compounds was measured by determining their level of decomposition through the use of liquid chromatography. The results are shown in Table 3.

Table 3

| Decomposition Rate of Compounds of Example 1 and Preparation Example | | |
|---|---|---|
| Storage period (50°C) | Decomposition Rate(%) | |
| | Test Compound | Amorphous Compound |
| 0 | 0 | 0 |
| 1 week | 0.4 | 4.2 |
| 2 week | 0.9 | 5.2 |

As can be readily seen from Tables 2 and 3, the crystalline hydrate of the formula(I) cephalosporin is much more stable than the compound in its amorphous form, with equal or better antibiotic effectiveness against a wide spectrum of pathogenic bacteria.

While the invention has been described with respect to the above specific embodiments, it should be recognized that various modifications and changes may be made within the scope of the invention as

defined by the claims that follow.

## Claims

1. A crystalline hydrate of 7-[(Z)-2-(2-aminothiazole-4-yl)-2-(2-carboxylprop-2-oxyimino)acetamido]-3-(5-methyl-1,4,6-triaminopirimidinium-2-yl)thiomethyl-3-cephem-4-carboxylate represented by formula(I):

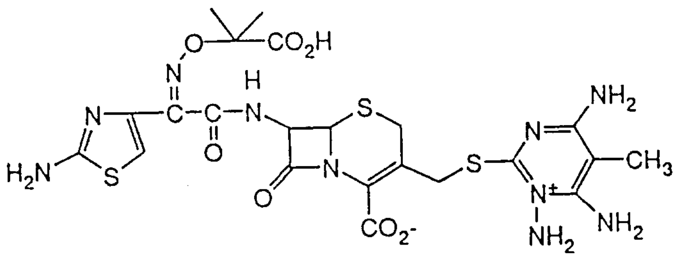

(I)

2. The crystalline hydrate of claim 1, which exits in one or more form of tri- to hepta-hydrate at a temperature ranging from 10 ° C to 60 ° C and a relative humidity ranging from 10 to 80%.

3. A process for preparing a crystalline hydrate of the compound defined in claim 1, which comprises the steps of:
   dissolving the compound in an amorphous form or its salt in an acidic or basic solution;
   adjusting the pH of the solution to a range from 1.5 to 5.5;
   precipitating the compound in a crystalline hydrate form in the solution; and
   recovering said crystalline hydrate of the compound.

4. The process of claims 3, wherein the pH is adjusted to a range from 2.5 to 4.0.

5. The process of claim 3, wherein said precipitation is carried out at a temperature ranging from 10 ° C to 60 ° C.

6. A pharmaceutical composition which comprises a therapeutically effective amount of the crystalline cephalosporin hydrate as defined in claim 1 as an active ingredient and a pharmaceutically acceptable carrier.

Figure 1

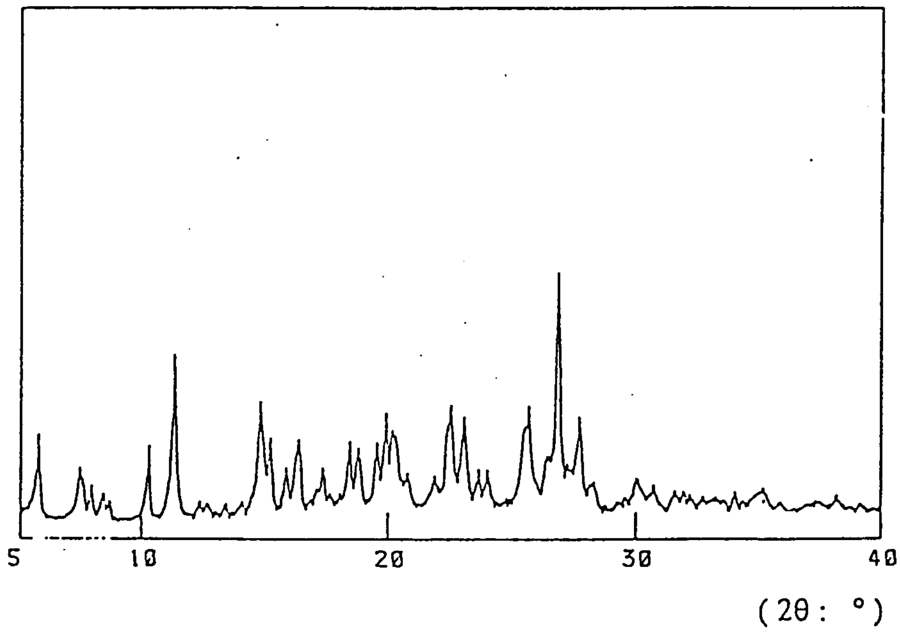


X-ray diffraction diagram (Cu-Kα, 32 KV, 15 mA)

European Patent Office

# EUROPEAN SEARCH REPORT

Application Number

EP 93 11 2822

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | EP-A-0 397 511 (LUCKY, LTD.) * page 24, compound I-34; table 1; pages 65, 66, example 49 * | 1,6 | C07D501/36 C07D501/12 |
| A | EP-A-0 449 515 (LUCKY, LTD.) * page 11, line 35 - page 15, line 30 * | 1 | |
| A | FR-A-2 585 705 (BRISTOL-MYERS CO.) * page 2, lines 28-35; pages 11, 12, example VII * | 1 | |
| A | EP-A-0 363 826 (MEIJI SEIKA KAISHA LTD.) * page 2, lines 31-51; page 4, lines 16-18 * | 1 | |
| A | EP-A-0 102 896 (RHONE-POULENC SANTE) * abstract; page 5, lines 1-3 * | 1 | |
| A | DE-A-2 949 485 (TAKEDA CHEMICAL INDUSTRIES, LTD.) * page 3; page 9, line 10 to page 10, line 6 * | 1 | TECHNICAL FIELDS SEARCHED (Int. Cl.5) |
| A | EP-A-0 033 518 (CIBA-GEIGY AG) * page 1, line 1 to page 2, line 7 * | 1 | C07D |

The present search report has been drawn up for all claims

| Place of search | Date of completion of the search | Examiner |
|---|---|---|
| BERLIN | 13 JANUARY 1994 | Christian Hass |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)